# EUROPÄISCHE PATENTSCHRIFT

(11) **EP 1 284 965 B1**
(45) Veröffentlichungstag und Bekanntmachung des Hinweises auf die Patenterteilung: **11.03.2009**
(21) Anmeldenummer: 01943202.0
(22) Anmeldetag: 29.03.2001
(51) Int. Cl.: C07D 209/88, B01J 19/00, C07B 37/10

(54) **VERFAHREN ZUR HERSTELLUNG VON INDOLEN**
METHOD FOR PRODUCING INDOLS
PROCEDE DE PREPARATION D'INDOLES

(30) Priorität: 29.05.2000 DE 10026646
(43) Veröffentlichungstag der Anmeldung: 26.02.2003
(73) Patentinhaber: Merck Patent GmbH, 64293 Darmstadt (DE)
(72) Erfinder: WURZIGER, Hanns, 64291 Darmstadt (DE); PIEPER, Guido, 64291 Darmstadt (DE); SCHWESINGER, Norbert, 98693 Ilmenau (DE)
(86) Internationale Anmeldenummer: PCT/EP2001/003573
(87) Internationale Veröffentlichungsnummer: WO 2001/092225

(56) Entgegenhaltungen:
- EP-A- 0 903 174
- WO-A-96/30113
- WO-A-99/22857
- DE-A- 2 425 227
- DE-A- 19 507 751
- US-A- 4 585 622
- LANCELOT J.-C.;GAZENGEL J.-M.; ROBBA M.: "Synthese de Dihydro-6,7 5H-pyrimidino[4,5-c]carbazoles et de 7H-Pyrimidino[5,4-c]carbazoles" CHEM. PHARM. BULL., Bd. 31, 1983, Seiten 2652-2661, XP001019058

## Beschreibung

Die vorliegende Erfindung betrifft ein Verfahren zur Herstellung von Indolen.

Die Herstellung von Indolen ist für die chemischen Industrie von großer Bedeutung, was sich sich auch in zahlreichen Veröffentlichungen zu diesem Thema widerspiegelt.

Die Herstellung von Indolen im technischen Maßstab bringt jedoch Sicherheitsprobleme und Gefahren mit sich. Zum einen werden häufig größere Mengen hochgiftige chemische Substanzen eingesetzt, die für sich allein bereits ein erhebliches Risiko für Mensch und Umwelt darstellen und zum anderen verlaufen Reaktionen zur Herstellung von Indolen häufig sehr stark exotherm, so daß bei der Durchführung dieser Reaktionen im technischen Maßstab eine erhöhte Explosionsgefahr besteht. Die Erlangung einer behördlichen Genehmigung nach dem BimschG für das Betreiben von Anlagen zur Herstellung von Indolen im technischen Maßstab ist daher mit einem beträchtlichen Aufwand verbunden.

Aufgabe der vorliegenden Erfindung ist es daher, ein Verfahren zur Herstellung von Indolen zur Verfügung zu stellen, das die oben genannten Nachteile vermeidet. Dieses Verfahren soll insbesondere in einfacher, reproduzierbarer Weise mit erhöhter Sicherheit für Mensch und Umwelt sowie mit guten Ausbeuten durchführbar sein und die Reaktionsbedingungen sollen sehr gut kontrollierbar sein.

Lancelot *et al.* offenbaren die Synthese von Dihydro-6,7 5*H*-pyrimidino[5,4-c]carbazoles und von 7*H*-Pyrimidino[5,4-c]carbazoles. Eine Synthese im Mikromischer wird nicht erwähnt (Lancelot et al., Chem. Pharm. Bull. 31(8)2652-2661 (1983).

Die DE 195 07 751 offenbart ein Verfahren zur Herstellung von substituierten 1,2,3,4-Tetrahydrocarbazolen. Eine Synthese im Mikromischer wird nicht erwähnt.

Mikromischer und ähnliche Vorrichtungen sind aus den nachfolgend genannten Dokumenten bekannt.

Die EP 0 903 174 offenbart ein Verfahren und Vorrichtung zur Oxidation organischer Verbindungen in flüssiger Phase unter Verwendung peroxidischer Oxidationsmittel.

Die US 4,585,622 offenbart einen Temperatur regulierten chemischen Mikroreaktor.

Die WO 96/30113 offenbart eine Vorrichtung zum Mischen kleiner Flüssigkeitsmengen.

Die W= 99/22857 offenbart ein Verfahren zur Durchführung einer chemischen Reaktion im Mikroreaktor.

Die DE 24 25 227 offenbart einen automatischen Mikroreaktor mit angeschlossenem Gaschromatographen zur Ausprüfung von Katalysatoren und zum Studium katalytischer Reaktionen unter Druck und Temperatur. Keines der vorstehend genannten Dokumente legt jedoch das erfindungsgemäße Verfahren zur Synthese von Indolen nahe.

Die Lösung dieser Aufgabe gelingt überraschenderweise durch das erfindungsgemäße Verfahren gemäß Anspruch 1.

Vorteilhafte Ausführungsformen des erfindungsgemäßen Verfahrens sind in den Unteransprüchen beschrieben.

Eine weitere bevorzugte Ausführungsform des erfindungsgemäßen Verfahrens ist dadurch gekennzeichnet, daß wenigstens ein Arylhydrazon in flüssiger oder gelöster Form in wenigstens einem temperierbaren Mikroreaktor erhitzt und durchmischt wird, während einer Verweilzeit reagiert und die so erhaltene Indolverbindung gegebenenfalls aus dem Reaktionsgemisch isoliert wird.

Erfindungsgemäß können einzelne Arylhydrazone oder Gemische aus mindestens zwei Arylhydrazonen nach dem beanspruchten Verfahren umgesetzt werden. Vorzugsweise wird jeweils nur ein Arylhydrazon nach dem erfindungsgemäßen Verfahren umgesetzt.

Ein Mikroreaktor im Sinne der Erfindung ist ein Reaktor mit einem Volumen ≤ 1000 µl in dem die Flüssigkeit(en) und/oder Lösung(en) wenigstens einmal innig vermischt werden. Vorzugsweise beträgt das Volumen des Mikroreaktors ≤ 100 µl, besonders bevorzugt ≤ 50 µl.

Der Mikroreaktor wird bevorzugt aus dünnen, miteinander verbundenen Siliziumstrukturen hergestellt.

Vorzugsweise ist der Mikroreaktor ein miniaturisierter Durchflußreaktor, besonders bevorzugt ein statischer Mikromischer. Ganz besonders bevorzugt ist der Mikroreaktor ein statischer Mikromischer, wie er in der Patentanmeldung mit der internationalen Veröffentlichungsnummer WO 96/30113 beschrieben ist, die hiermit als Referenz eingeführt wird und als Teil der Offenbarung gilt. Ein solcher Mikroreaktor weist kleine Kanäle auf, in denen Flüssigkeiten und/oder in Lösungen vorliegende, chemische Verbindungen durch die kinetische Energie der strömenden Flüssigkeiten und/oder Lösungen miteinander vermischt werden.

Die Kanäle des Mikroreaktors weisen vorzugsweise einen Durchmesser von 10 bis 1000 µm, besonders bevorzugt von 20 bis 800 µm und ganz besonders bevorzugt von 30 bis 400 µm auf.

Vorzugsweise wird/werden die Flüssigkeit(en) und/oder Lösung(en) so in den Mikroreaktor gepumpt, daß sie diesen mit einer Durchflußgeschwindigkeit von 0,01 µl/min bis 100 ml/min, besonders bevorzugt 1 µl/min bis 1 ml/min durchströmen/durchströmt.

Erfindungsgemäß ist der Mikroreaktor vorzugsweise über einen Auslaß mit wenigstens einer Verweilstrecke, vorzugsweise einer Kapillare, besonders bevorzugt einer temperierbaren Kapillare verbunden. In diese Verweilstrecke bzw. Kapillare werden die Flüssigkeiten und/oder Lösungen nach ihrer Durchmischung im Mikroreaktor zur Verlängerung ihrer Verweilzeit geführt.

Die Verweilzeit im Sinne der Erfindung ist die Zeit zwischen der Durchmischung der Edukte und der Aufarbeitung der resultierenden Reaktionslösung zur Analyse bzw. Isolierung der (des) gewünschten Produkte(s).

Die erforderliche Verweilzeit bei dem erfindungsgemäßen Verfahren hängt von verschiedenen Parametern ab, wie z.B. der Temperatur oder der Reaktivität der Edukte. Dem Fachmann ist es möglich, die Verweilzeit an diese Parameter anzupassen und so einen optimalen Reaktionsverlauf zu erzielen.

Die Verweilzeit der Reaktionslösung in dem zum Einsatz kommenden System aus wenigstens einem Mikroreaktör und gegebenenfalls einer Verweilstrecke kann durch die Wahl der Durchflußgeschwindigkeit der eingesetzten Flüssigkeit(en) und/oder Lösung(en) eingestellt werden.

Ebenfalls bevorzugt wird das Reaktionsgemisch durch zwei oder mehr in Reihe geschaltete Mikroreaktoren geführt. Hierdurch wird erreicht, daß auch bei erhöhter Durchflußgeschwindigkeit die Verweilzeit verlängert wird und die eingesetzten Komponenten so umgesetzt werden, daß eine optimale Produktausbeute des/der gewünschten Indols/Indole erreicht wird.

In einer weiteren bevorzugten Ausführungsform wird das Reaktionsgemisch durch zwei oder mehr parallel angeordnete Mikroreaktoren geleitet, um den Durchsatz zu erhöhen.

In einer anderen bevorzugten Ausführungsform des erfindungsgemäßen Verfahrens wird die Zahl und die Anordnung der Kanäle in einem oder mehreren Mikroreaktor(en) so variiert, daß die Verweilzeit verlängert wird, so daß auch hier bei erhöhter Durchflußgeschwindigkeit eine optimale Ausbeute an dem/den gewünschten Indol(en) erreicht wird.

Vorzugsweise beträgt die Verweilzeit der Reaktionslösung im Mikroreaktor, gegebenfalls im Mikroreaktor und der Verweilstrecke ≤ 15 Stunden, vorzugsweise ≤ 3 Stunden, besonders bevorzugt ≤ 1 Stunde.

Das erfindungsgemäße Verfahren kann in einem sehr breiten Temperaturbereich durchgeführt werden, der im wesentlichen durch die Temperaturbeständigkeit der zum Bau des Mikroreaktors, gegebenenfalls der Verweilstrecke, sowie weiterer Bestandteile, wie z.B. Anschlüsse und Dichtungen eingesetzten Materialien und durch die physikalischen Eigenschaften der eingesetzten Lösungen und/oder Flüssigkeiten beschränkt ist.

Sofern das erfindungsgemäße Verfahren in Gegenwart eines Katalysators durchgeführt wird, wird es vorzugsweise bei einer Temperatur im Bereich von - 100 bis +250 °C, besonders bevorzugt im Bereich von -78 bis +150°C und ganz besonders bevorzugt im Bereich von 0 bis +40 °C durchgeführt.

Wird das erfindungsgemäße Verfahren ohne einen Katalysator durchgeführt, so wird es vorzugsweise bei einer Temperatur im Bereich von 80 bis 200 °C, besonders bevorzugt im Bereich von 90 bis 150 °C und ganz besonders bevorzugt im Bereich von 100 bis 120 °C durchgeführt.

Das erfindungsgemäße Verfahren kann sowohl kontinuierlich als auch diskontinuierlich durchgeführt werden. Vorzugsweise wird es kontinuierlich durchgeführt.

Für die Durchführung des erfindungsgemäßen Verfahrens zur Herstellung von Indolen ist es erforderlich, daß die Reaktion möglichst in homogener flüssiger Phase, die keine oder nur sehr kleine Feststoffpartikel enthält, durchgeführt wird, da sonst die in den Mikroreaktoren vorhandenen Kanäle verstopft werden.

Der Reaktionsverlauf kann bei dem erfindungsgemäßen Verfahren zur Herstellung von Indolen mit verschiedenen, dem Fachmann bekannten analytischen Methoden verfolgt und gegebenenfalls geregelt werden. Vorzugsweise wird der Reaktionsverlauf chromatographisch, besonders bevorzugt durch Hochdruckflüssigkeitschromatographie verfolgt und gegebenenfalls geregelt. Die Kontrolle der Reaktion ist dabei im Vergleich zu bekannten Verfahren deutlich verbessert.

Nach der Reaktion wird/werden das/die Indol(e) gegebenenfalls isoliert. Vorzugsweise wird/werden das/die Indol(e) durch Extraktion aus dem Reaktionsgemisch isoliert.

Als Arylhydrazone können bei dem erfindungsgemäßen Verfahren alle dem Fachmann als Substrate zur Herstellung von Indolen bekannten Arylhydrazone eingesetzt werden.

In einer bevorzugten Ausführungsform des erfindungsgemäßen Verfahrens wird wenigstens ein Arylhydrazon eines organischen Ketons oder eines organischen Aldehydes, besonders bevorzugt ein Arylhydrazon eines aliphatischen, aromatischen oder heteroaromatischen Ketons oder ein Arylhydrazon eines aliphatischen, aromatischen oder heteroaromatischen Aldehydes eingesetzt.

Als Arylhydrazone eines aliphatischen Ketons oder eines aliphatischen Aldehyds können alle dem Fachmann bekannten Arylhydrazone von aliphatischen Ketonen oder aliphatischen Aldehyden eingesetzt werden, die sich als Substrate zur Herstellung von Indolen eignen. Dabei sind auch Arylhydrazone von geradkettigen, verzweigten, cyclischen, gesättigten und ungesättigten Ketonen und/oder Arylhydrazone von geradkettigen, verzweigten, cyclischen, gesättigten und ungesättigten Aldehyden umfaßt.

Als Arylhydrazone eines aromatisches Ketons oder eines aromatischen Aldehyds können alle dem Fachmann bekannten Arylhydrazone von aromatischen Ketonen und/oder aromatischen Aldehyden eingesetzt werden, die sich als Substrate zur Herstellung von Indolen eignen. Im Sinne der Erfindung werden damit Arylhydrazone von aromatischen Aldehyden und Ketonen umfaßt, die ein monocyclisches und/oder polycyclisches homoaromatisches Grundgerüst oder eine entsprechende Teilstruktur, z.B. in Form von Substituenten, aufweisen.

Als Arylhydrazone eines heteroaromatischen Ketons oder eines heteroaromatisches Aldehyds können alle dem Fachmann bekannten Arylhydrazone von heteroaromatischen Ketonen oder heteroaromatischen Aldehyden eingesetzt werden, die sich als Substrate zur Herstellung von Indolen eignen und wenigstens ein Heteroatom enthalten. Arylhydrazone von heteroaromatischen Ketonen oder heteroaromatischen Aldehyden im Sinne der Erfindung umfassen Arylhydrazone von heteroaromatischen Ketonen oder Aldehyden, die wenigstens ein monocyclisches und/oder polycyclisches heteroaromatisches Grundgerüst oder eine entsprechende Teilstruktur, z.B. in Form von Substituenten, aufweisen. Diese heteroaromatischen Grundgerüste oder Teilstrukturen weisen bevorzugt wenigstens ein Sauerstoff-, Stickstoff-und/oder Schwefelatom auf.

Die Arylhydrazone der aliphatischen, aromatischen oder heteroaromatischen Ketone und Aldehyde lassen sich nach den üblichen, dem Fachmann an sich bekannten Methoden herstellen. Vorzugsweise werden die Arylhydrazone durch Kondensation der entsprechenden Arylhydrazine mit den entsprechenden aliphatischen, aromatischen oder heteroaromatischen Ketonen bzw. Aldehyden hergestellt.

In einer weiteren bevorzugten Ausführungsform des erfindungsgemäßen Verfahrens wird als Arylhydrazon ein Phenylhydrazon, besonders bevorzugt ein Phenylhydrazon eines aliphatischen, aromatischen oder heteroaromatischen Ketons oder Aldehyds eingesetzt.

In einer weiteren bevorzugten Ausführungsform des erfindungsgemäßen Verfahrens wird das eingesetzte Arylhydrazon des aliphatischen, aromatischen oder heteroaromatischen Ketons und/oder des aliphatischen, aromatischen oder heteroaromatischen Aldehyds in situ, vorzugsweise aus dem entsprechenden Arylhydrazin und dem entsprechenden aliphatischen, aromatischen oder heteroaromatischen Keton und/oder dem entsprechenden aliphatischen, aromatischen oder heteroaromatischen Aldehyd, in wenigstens einem Mikromischer gebildet. In-situ-Bildung im Sinne der vorliegenden Erfindung bedeutet, daß das Arylhydrazon unmittelbar vor der Umsetzung zu dem entsprechenden Indol gebildet wird.

Ebenfalls bevorzugt kann das Arylhydrazon, vorzugsweise aus dem entsprechenden Arylhydrazin und dem entsprechenden aliphatischen, aromatischen oder heteroaromatischen Keton und/oder dem entsprechenden aliphatischen, aromatischen oder heteroaromatischen Aldehyd, in wenigstens einem Mikromischer gebildet und vor der Umsetzung zu dem entsprechenden Indol isoliert werden.

Als Katalysatoren können bei dem erfindungsgemäßen Verfahren sämtliche, dem Fachmann bekannten, zur Herstellung von Indolen geeigneten Katalysatoren oder eine Mischung aus mindestens zwei dieser Katalysatoren eingesetzt werden. Vorzugsweise wird jeweils nur ein Katalysator eingesetzt.

In einer weiteren bevorzugten Ausführungsform der vorliegenden Erfindung wird als Katalysator eine anorganische Säure, eine organische Säure, eine

Lewissäure oder ein Gemisch aus wenigstens zwei dieser Katalysatoren eingesetzt.

Als anorganische Säure kann bevorzugt Schwefelsäure, Salzsäure, Perchlorsäure, (Poly-)phosphorsäure, Trifluoressigsäure, Salpetersäure oder ein Gemisch aus wenigstens zwei dieser anorganischen Säuren eingesetzt werden.

Als organische Säure kann vorzugsweise Chlorsulfonsäure, p-Toluolsulfonsäure, Methansulfonsäure, Trifluormethansulfonsäure oder ein Gemisch aus wenigstens zwei dieser organischen Säuren eingesetzt werden.

Als Lewissäure kann vorzugsweise eine Borhalogenverbindung, besonders bevorzugt BF₃, ein Metallhalogenid, besonders bevorzugt ZnCl₂, SnCl₄, AlCl₃, FeCl₃, TiCl₄, MgCl₂, ganz besonders bevorzugt ZnCl₂, oder ein Gemisch aus wenigstens zwei dieser Lewis-Säuren eingesetzt werden.

In einer weiteren bevorzugten Ausführungsform werden zwischen 0,1 und 110 Mol-%, besonders bevorzugt zwischen 1 und 100 Mol-%, ganz besonders bevorzugt zwischen 10 und 50 Mol-% des/der Katalysators/Katalysatoren, bezogen auf die eingesetzte Menge an Arylhydrazon(en) eingesetzt.

Für das erfindungsgemäße Verfahren ist es wesentlich, daß die eingesetzten Arylhydrazone und ggf. die Katalysatoren entweder selbst flüssig sind oder in gelöster Form vorliegen. Sofern die eingesetzten Arylhydrazone oder die Katalysatoren selbst flüssig sind, können sie ggf. auch selbst als Lösungsmittel für weitere Komponenten der Reaktion eingesetzt werden. Sofern diese nicht schon selbst in flüssiger Form vorliegen, müssen sie vor der Durchführung des erfindungsgemäßen Verfahrens in einem geeigneten Lösungsmittel gelöst werden. Als Lösungsmittel werden bevorzugt halogenierte Lösungsmittel, besonders bevorzugt Dichlormethan, Chloroform, 1,2-Dichlorethan oder 1,1,2,2-Tetrachlorethan, geradkettige, verzweigte oder cyclische Paraffine, besonders bevorzugt Pentan, Hexan, Heptan, Octan, Cyclopentan, Cyclohexan, Cycloheptan oder Cyclooctan oder geradkettige, verzweigte oder cyclische Ether, besonders bevorzugt Diethylether, Methyl-tert.-butylether, Tetrahydrofuran oder Dioxan, aromatische Lösungsmittel, besonders bevorzugt Toluol, Xylole, Ligroin oder Phenylether, N-haltige heterocyclische Lösungsmittel, besonders bevorzugt Pyridin oder N-Methylpyrrolidon, oder ein Gemisch aus wenigstens zwei dieser Lösungsmittel eingesetzt.

Bei dem erfindungsgemäßen Verfahren ist die Gefahr für Mensch und Umwelt durch austretende Chemikalien erheblich verringert und führt somit zu einer erhöhten Sicherheit beim Umgang mit Gefahrstoffen. Die Herstellung von Indolen nach dem erfindungsgemäßen Verfahren ermöglicht ferner eine bessere Kontrolle der Reaktionsbedingungen, wie z.B. Reaktionsdauer und Reaktionstemperatur, als dies in den herkömmlichen Verfahren möglich ist. Weiterhin ist bei dem erfindungsgemäßen Verfahren die Gefahr von Explosionen bei sehr stark exothermen Reaktionen zur Herstellung von Indolen deutlich vermindert. Die Temperatur kann in jedem Volumenelement des Systems individuell gewählt und konstant gehalten werden. Der Reaktionsverlauf ist bei dem erfindungsgemäßen Verfahren zur Herstellung von Indolen sehr schnell und genau regelbar. Die Indole lassen sich so in sehr guten und reproduzierbaren Ausbeuten erhalten.

Besonders vorteilhaft ist auch, daß das erfindungsgemäße Verfahren kontinuierlich durchgeführt werden kann. Hierdurch ist es im Vergleich zu herkömmlichen Verfahren schneller und kostengünstiger und es ist ohne großen Meß- und Regelungsaufwand möglich, beliebige Mengen der Indole herzustellen.

Im folgenden wird die Erfindung anhand eines Beispiels erläutert. Dieses Beispiel dient lediglich der Erläuterung der Erfindung und schränkt den allgemeinen Erfindungsgedanken nicht ein.

### Beispiel

### Herstellung von 1,2,3,4-Tetrahydrocarbazol-4-on aus Phenylhydrazin und 1,3-Cyclohexandion

Die Herstellung von 1,2,3,4-Tetrahydrocarbazol-4-on aus Phenylhydrazin und 1,3-Cyclohexandion erfolgte in einem statischen Mikromischer (Technische Universität Ilmenau, Fakultät Maschinenbau, Dr.-Ing. Norbert Schwesinger, Postfach 100565, D-98684, Ilmenau) mit einer Baugröße von 40 mm x 25 mm x 1 mm, der insgesamt 11 Mischstufen mit einem Volumen von jeweils 0,125 µl aufwies. Der Gesamtdruckverlust betrug circa 1000 Pa.

Der statische Mikromischer war über einen Auslaß und eine Omnifit Mitteldruck-HPLC-Verbindungskomponente (Omnifit, Großbritannien) an eine Teflon-Kapillare mit einem Innendurchmesser von 0,49 mm und einer Länge von 1,0 m verbunden. Die Reaktion wurde bei 100 °C durchgeführt. Der statische Mikromischer und die Teflon-Kapillare wurden dazu in einem thermostatisierten Doppelmantelgefäß temperiert.

Es wurde eine 2 ml Einweginjektionsspritze mit einem Teil einer Lösung aus 1,0 g (10 mmol) Phenylhydrazin in 40,5 ml 50 %-iger Schwefelsäure und eine weitere 2 ml Einwegspritze mit einem Teil einer Lösung aus 1,1 g (10 mmol) 1,3-Cyclohexandion in 40,5 ml 50 %-iger Schwefelsäure befüllt. Anschließend wurde der Inhalt beider Spritzen mit einer Dosierpumpe (Harvard Apparatus Inc., Pump 22, South Natick, Massachussets, USA) in den statischen Mikromischer überführt.

Die Versuchsanordnung wurde vor der Durchführung der Reaktion in Bezug auf die Abhängigkeit der Verweilzeit von der Pumpenflußrate kalibriert. Die Verweilzeit wurde auf 1; 2,5; 5; 10; 15; 20; 25; 30; und 60 Minuten eingestellt. Die Reaktionen wurden mit Hilfe eines Merck Hitachi LaChrom HPLC-Instruments verfolgt. Auch das den jeweiligen Reaktionsbedingungen und Verweilzeiten entsprechende Verhältnis von Edukt zu Produkt wurde mittels HPLC auf dem vorstehend genannten Instrument bestimmt.

## Patentansprüche

1. Verfahren zur Herstellung von Indolen, wobei
a) ein flüssiges oder in einem Lösungsmittel gelöstes Arylhydrazon eines organischen Aldehyds oder eines organischen Ketons
mit
b) wenigstens einem flüssigen oder in einem Lösungsmittel gelösten Katalysator
wenigstens einmal innig vermischt wird,
**dadurch gekennzeichnet, dass** nach der Vermischung eine Verweilzeit eingehalten wird, während derer kein Edukt und/oder Katalysator zugefügt wird, und dass das Verfahren in wenigstens einem Mikroreaktor in Form eines statischen Mikromischers mit einem Volumen von ≤ 1000 in homogener flüssiger Phase, die keine Feststoffpartikel enthält, durchgeführt wird.

2. Verfahren gemäß Anspruch 1, **dadurch gekennzeichnet, dass** der Mikroreaktor über einen Auslaß mit einer Kapillare verbunden ist.

3. Verfahren nach einem der Ansprüche 1 oder 2, **dadurch gekennzeichnet, dass** als Arylhydrazon ein Phenylhydrazon eingesetzt wird.

4. Verfahren gemäß einem oder mehreren der Ansprüche 1 - 3, **dadurch gekennzeichnet, dass** die durch die Reaktion gebildete Indolverbindung aus dem Reaktionsgemisch isoliert wird.

5. Verfahren nach einem oder mehreren der Ansprüche 1 bis 4, **dadurch gekennzeichnet, dass** das gebildete Indol durch Extraktion aus dem Reaktionsgemisch isoliert wird.

6. Verfahren nach einem oder mehreren der Ansprüche 1 - 5, **dadurch gekennzeichnet, dass** als Katalysator eine anorganische Säure, eine organische Säure, eine Lewissäure, oder ein Gemisch aus wenigstens zwei dieser Katalysatoren eingesetzt wird.

7. Verfahren nach Anspruch 6, **dadurch gekennzeichnet, dass** als anorganische Säure Schwefelsäure, Salzsäure, Perchlorsäure, (Poly-)phosphorsäure, Trifluoressigsäure, Salpetersäure oder ein Gemisch aus wenigstens zwei dieser anorganischen Säuren eingesetzt wird.

8. Verfahren nach Anspruch 6, **dadurch gekennzeichnet, dass** als organische Säure Chlorsulfonsäure, p-Toluolsulfonsäure, Methansulfonsäure, Trifluormethansulfonsäure oder ein Gemisch aus wenigstens zwei dieser organischen Säuren eingesetzt wird.

9. Verfahren nach Anspruch 6, **dadurch gekennzeichnet, dass** als Lewissäure eine Borhalogenverbindung, vorzugsweise BF₃, oder ein Metallhalogenid, oder ein Gemisch aus wenigstens zwei dieser Lewis-Säuren eingesetzt wird.

10. Verfahren nach einem oder mehreren der Ansprüche 1 bis 9, **dadurch gekennzeichnet, dass** zwischen 0,1 und 110 Mol-%, des/der Katalysators/Katalysatoren bezogen auf die eingesetzte Menge an Arylhydrazon(en) eingesetzt werden.

11. Verfahren nach Anspruch 10, **dadurch gekennzeichnet, dass** es bei einer Temperatur im Bereich von -100 bis +250 °C durchgeführt wird.

12. Verfahren nach einem oder mehreren der Ansprüche 1 bis 11, **dadurch gekennzeichnet, dass** der Mikroreaktor ein miniaturisierter Durchflußreaktor ist, der Kanäle mit einem Durchmesser von 10 bis 1000 µm aufweist.

13. Verfahren nach einem oder mehreren der Ansprüche 1 bis 12, **dadurch gekennzeichnet, dass** das Volumen des Mikroreaktors ≤ 100 µl beträgt.

14. Verfahren nach einem oder mehreren der Ansprüche 1 bis 13, **dadurch gekennzeichnet, dass** das Reaktionsgemisch den Mikroreaktor mit einer Durchflußgeschwindigkeit von 0,01 µl/min bis 100 ml/min durchströmt.

15. Verfahren nach einem oder mehreren der Ansprüche 1 bis 13, **dadurch gekennzeichnet, dass** die Verweilzeit der eingesetzten Verbindungen im Mikroreaktor, gegebenfalls im Mikroreaktor und der Kapillaren ≤ 15 Stunden beträgt.

16. Verfahren nach einem oder mehreren der Ansprüche 1 bis 15, **dadurch gekennzeichnet, dass** der Reaktionsverlauf chromatographisch verfolgt und gegebenenfalls geregelt wird.

17. Verfahren nach einem oder mehreren der Ansprüche 1 bis 16, **dadurch gekennzeichnet, dass** das Arylhydrazon, vorzugsweise aus dem entsprechenden Arylhydrazin und dem entsprechenden aliphatischen, aromatischen oder heteroaromatischen Keton und/oder dem entsprechenden aliphatischen, aromatischen oder heteroaromatischen Aldehyd, in wenigstens einem Mikromischer gebildet und vor der Umsetzung zu dem entsprechenden Indol isoliert wird.

## Claims

1. Process for the preparation of indoles, in which
a) a liquid or solvent-dissolved arylhydrazone of an organic aldehyde or of an organic ketone
is mixed intimately at least once with
b) at least one liquid or solvent-dissolved catalyst,
**characterised in that**, after the mixing, a residence time is observed during which no starting material and/or catalyst is added, and **in that** the process is carried out in at least one microreactor in the form of a static micromixer having a capacity of ≤ 1000 µl in the homogeneous liquid phase which comprises no solids particles.

2. Process according to Claim 1, **characterised in that** the microreactor is connected via an outlet to a capillary.

3. Process according to one of Claims 1 or 2, **characterised in that** the arylhydrazone employed is a phenylhydrazone.

4. Process according to one or more of Claims 1 - 3, **characterised in that** the indole compound formed by the reaction is isolated from the reaction mixture.

5. Process according to one or more of Claims 1 to 4, **characterised in that** the indole formed is isolated from the reaction mixture by extraction.

6. Process according to one or more of Claims 1 - 5, **characterised in that** the catalyst employed is an inorganic acid, an organic acid, a Lewis acid, or a mixture of at least two of these catalysts.

7. Process according to Claim 6, **characterised in that** the inorganic acid employed is sulfuric acid, hydrochloric acid, perchloric acid, (poly)phosphoric acid, trifluoroacetic acid, nitric acid or a mixture of at least two of these inorganic acids.

8. Process according to Claim 6, **characterised in that** the organic acid employed is chlorosulfonic acid, p-toluenesulfonic acid, methanesulfonic acid, trifluoromethanesulfonic acid or a mixture of at least two of these organic acids.

9. Process according to Claim 6, **characterised in that** the Lewis acid employed is a boron-halogen compound, preferably BF₃, or a metal halide, or a mixture of at least two of these Lewis acids.

10. Process according to one or more of Claims 1 to 9, **characterised in that** between 0.1 and 110 mol% of the catalyst(s) are employed, based on the amount of arylhydrazone(s) employed.

11. Process according to Claim 10, **characterised in that** it is carried out at a temperature in the range from -100 to +250°C.

12. Process according to one or more of Claims 1 to 11, **characterised in that** the microreactor is a miniaturised flow reactor which has channels having a diameter of 10 to 1000 µm.

13. Process according to one or more of Claims 1 to 12, **characterised in that** the capacity of the microreactor is ≤ 100 µl.

14. Process according to one or more of Claims 1 to 13, **characterised in that** the reaction mixture flows through the microreactor at a flow rate of 0.01 µl/min to 100 ml/min.

15. Process according to one or more of Claims 1 to 13, **characterised in that** the residence time of the compounds employed in the microreactor, where appropriate in the microreactor and the capillaries, is ≤ 15 hours.

16. Process according to one or more of Claims 1 to 15, **characterised in that** the course of the reaction is monitored by chromatography and regulated if appropriate.

17. Process according to one or more of Claims 1 to 16, **characterised in that** the arylhydrazone is formed in at least one micromixer, preferably from the corresponding arylhydrazine and the corresponding aliphatic, aromatic or heteroaromatic ketone and/or the corresponding aliphatic, aromatic or heteroaromatic aldehyde, and isolated before conversion into the corresponding indole.

## Revendications

1. Procédé de préparation d'indoles, dans lequel
a) une arylhydrazone, liquide ou solubilisée dans un solvant, d'un aldéhyde organique ou d'une cétone organique
est mélangée de manière intime au moins une fois avec
b) au moins un catalyseur liquide ou solubilisé dans un solvant,
**caractérisé en ce que**, après le mélange, on respecte un temps de séjour pendant lequel on n'ajoute aucun produit de départ et/ou catalyseur, et **en ce que** le procédé est effectué dans au moins un microréacteur sous forme d'un micro-mélangeur statique ayant une capacité ≤ 1000 µl dans la phase liquide homogène qui ne comprend pas de particules de matières solides.

2. Procédé selon la revendication 1, **caractérisé en ce que** le microréacteur est relié à un capillaire par l'intermédiaire d'un orifice de sortie.

3. Procédé selon l'une des revendications 1 ou 2, **caractérisé en ce que** l'arylhydrazone employée est une phénylhydrazone.

4. Procédé selon l'une ou plusieurs parmi les revendications 1 - 3, **caractérisé en ce que** le composé d'indole formé par la réaction est isolé à partir du mélange réactionnel.

5. Procédé selon l'une ou plusieurs parmi les revendications 1 à 4, **caractérisé en ce que** l'indole formé est isolé à partir du mélange réactionnel par extraction.

6. Procédé selon l'une ou plusieurs parmi les revendications 1 - 5, **caractérisé en ce que** le catalyseur employé est un acide minéral, un acide organique, un acide de Lewis, ou un mélange d'au moins deux parmi ces catalyseurs.

7. Procédé selon la revendication 6, **caractérisé en ce que** l'acide minéral employé est l'acide sulfurique, l'acide chlorhydrique, l'acide perchlorique, l'acide (poly)phosphorique, l'acide trifluoroacétique, l'acide nitrique ou un mélange d'au moins deux parmi ces acides minéraux.

8. Procédé selon la revendication 6, **caractérisé en ce que** l'acide organique employé est l'acide chlorosulfonique, l'acide p-toluènesulfonique, l'acide méthanesulfonique, l'acide trifluorométhanesulfonique ou un mélange d'au moins deux parmi ces acides organiques.

9. Procédé selon la revendication 6, **caractérisé en ce que** l'acide de Lewis employé est un composé de bore-halogène, préférablement BF₃, ou un halogénure de métal, ou un mélange d'au moins deux parmi ces acides de Lewis.

10. Procédé selon l'une ou plusieurs parmi les revendications 1 à 9, **caractérisé en ce qu'**entre 0,1 et 110% molaire du ou des catalyseurs sont employés, sur la base de la quantité d'arylhydrazone(s) employée.

11. Procédé selon la revendication 10, **caractérisé en ce qu'**il est effectué à une température dans la plage allant de -100 à +250°C.

12. Procédé selon l'une ou plusieurs parmi les revendications 1 à 11, **caractérisé en ce que** le microréacteur est un réacteur continu miniaturisé ayant des canaux d'un diamètre allant de 10 à 1000 µm.

13. Procédé selon l'une ou plusieurs parmi les revendications 1 à 12, **caractérisé en ce que** la capacité du microréacteur est ≤ 100 µl.

14. Procédé selon l'une ou plusieurs parmi les revendications 1 à 13, **caractérisé en ce que** le mélange réactionnel s'écoule à travers le microréacteur à un débit allant de 0,01 µl/min à 100 ml/min.

15. Procédé selon l'une ou plusieurs parmi les revendications 1 à 13, **caractérisé en ce que** le temps de séjour des composés employés dans le microréacteur, le cas échéant dans le microréacteur et les capillaires, est ≤ 15 heures.

16. Procédé selon l'une ou plusieurs parmi les revendications 1 à 15, **caractérisé en ce que** le déroulement de la réaction est suivi par chromatographie et régulé le cas échéant.

17. Procédé selon l'une ou plusieurs parmi les revendications 1 à 16, **caractérisé en ce que** l'arylhydrazone est formée dans au moins un micro-mélangeur, préférablement à partir de l'arylhydrazine correspondante et de la cétone aliphatique, aromatique ou hétéroaromatique correspondante et/ou de l'aldéhyde aliphatique, aromatique ou hétéroaromatique correspondant, et isolée avant conversion en indole correspondant.
